# EUROPEAN PATENT APPLICATION

(11) **EP 4 550 341 A1**
(43) Date of publication of application: **07.05.2025**
(21) Application number: 23207492.2
(22) Date of filing: 02.11.2023
(51) Int. Cl.: G16H 10/40, G16H 40/20, G16H 40/40, G16H 40/67, G01N 35/00, G06Q 10/20

(54) **REMOTE ANALYZER MONITORING**

(71) Applicant: Roche Diagnostics International AG, 6343 Rotkreuz (CH); Roche Diagnostics Operations, Inc., Indianapolis, Indiana 46250 (US)
(72) Inventor: ALEXANDER, Jeffrey, Indianapolis, IN 46256-1025 (US); HOOGENDOORN, Geertje Engelina Adriana Maria, 6343 Rotkreuz ZG (CH); LIN, Yi-Chun, Indianapolis, IN 46256-1025 (US); WINIARZ, Jakub, 6343 Rotkreuz ZG (CH)
(74) Representative: Peterreins Schley

(57) **Abstract**

A computer implemented method (60) for remote analyzer monitoring, comprising: obtaining (62), via a computing device (20) comprising a camera (21), visual representation data (70) of at least a display interface (P1-D) of an automated analyzer (PI) after the automated analyzer (PI) has performed a predefined operation, wherein the visual representation data (70) of the display interface (P1-D) comprises data associated with an outcome of the predefined operation performed by the automated analyzer (P1);
processing (64) the visual representation data to extract data (74) relating to an outcome of the predefined operation computed by the automated analyzer (P1) and comprised in the visual representation data (70) associated with the predefined operation;
evaluating (66) the data associated with the predefined operation according to at least one evaluation criterion to thus generate evaluation data (76); and
storing (68) the evaluation data associated with the predefined operation.

## Description

### Technical Field

This disclosure relates to a computer implemented method for remote automated analyzer monitoring, and an associated apparatus, system, and computer program element.

### Background

In clinical care environments, automated analyzers of medical samples can be used at, or near to, the point of care. Such automated analyzers, or analytical devices, are designated "Point of Care (POC) testing devices." A wide variety of automated analyzers often coexist in the same medical care facility.

To facilitate the management of such automated analyzers, a point of care device management system and/or remotely accessible POC management software enables the monitoring of automated analyzers. The point of care device management system may, for example, obtain and track patient results, log inventory and maintenance issues, monitor the certification of users, and monitor the success of quality control procedures applied regularly to the automated analyzers.

The management software provided for use in a point of care device management system is often effective in respect of more modem automated analyzers. However, many health care facilities are reluctant to change older, or legacy, automated analyzers which still function acceptably well. The legacy automated analyzers are often not integrated, with modem POC management software. Alternatively, integrating the legacy automated analyzers with modem POC management software may require custom software modules to be laboriously coded for a single healthcare facility. In view of the difficulties of validating and certifying such software modules for use in a healthcare facility, such custom coding is not often attempted. Therefore, the presence of even one legacy automated analyzer at a healthcare facility often implies recourse to paper- based and non- automated workarounds for analyzer monitoring.

Accordingly, approaches for integrating legacy automated analyzers into modem POC management software can be further improved.

### Summary

According to a first aspect, there is provided a computer implemented method for remote automated analyzer monitoring, comprising obtaining, via a computing device comprising a camera, visual representation data of at least a display interface of an automated analyzer after the automated analyzer has performed a predefined operation, wherein the visual representation data of the display interface comprises data associated with an outcome of the predefined operation performed by the automated analyzer. The computer implemented method further comprises processing the visual representation data to extract data relating to an outcome of the predefined operation computed by the automated analyzer and comprised in the visual representation data associated with the predefined operation. The computer implemented method further comprises evaluating the data associated with the predefined operation according to at least one evaluation criterion to thus generate evaluation data. The computer implemented method further comprises storing the evaluation data associated with the predefined operation.

According to an embodiment, the remote automated analyzer monitoring is performed via a side channel.

An effect is that the result of a predetermined operation performed on a legacy automated analyzer is captured and provided to a remote data processing agent. For example, the result of the predetermined operation performed on the legacy automated analyzer is communicated via side channel to the remote data processing agent. In other words, the result of the predetermined operation is communicated to the data processing agent on a channel that is not identical to a channel used to communicate native messages from the legacy automated analyzer. In other words, the capture of visual representation data of a display after the predefined operation has been performed using the legacy automated analyzer, avoids the need for an inconvenient firmware upgrade of the legacy analyzer to be performed. Because the remote data processing agent is capable of capturing any element displayed on the screen of the legacy automated analyzer, additional functionality and additional data can be provided to the remote data processing agent that is not available to the signalling of the legacy automated analyzer. For example, the display may provide extra information that is not available from the signalling schema of a legacy device.

In some cases, the use of the legacy automated analyzer with the software configured to report visual information data to the remote data processing agent extends the in- service life of the legacy automated analyzer, because the need to replace the legacy automated analyzer with an automated analyzer capable of communicating with the remote processing agent through a direct communication channel (as opposed to a side communication channel) is removed. Therefore, the waste of a functional analyzer with an obsolete communication function can be avoided.

Accordingly, it is easier to report the technical condition of the legacy automated analyzer. In an embodiment, the computer comprising a camera can execute an application requiring user authentication of the application. Accordingly, the visual representation data is logically linked to the user authentication of the application, enabling the remote data processing agent to effectively authenticate the visual representation data obtained from the legacy analyzer to a staff member (and thus, their certification level).

Some legacy analyzers may lack a communication interface and be used as stand-alone analyzers. In this case, the technique may enable the reporting of data from the legacy analyzer to a POC-DMS, where such communication was not possible before. Image analysis to extract result data at a modem server (in a process similar to optical character recognition) may, in many examples, consume much less engineering development time as compared to writing new embedded software code to interface with legacy drivers of a legacy automated analyzer.

Metadata (such as location data, or user data of a user of the computer comprising a camera) accessible to a computing device taking a photograph or video of a legacy automated analyzer can be added to the photograph or video transmitted to the data processing agent, again enabling the visual representation data to contain additional information about, for example, a user of the legacy analyzer that cannot be transmitted in the legacy signalling schema.

In other words, in some examples a decentralized, wireless, photographically based governance of remote analytical devices is enabled from a central location without the need to connect to the analytical devices from a legacy communication network. By registering the application and corresponding instruments in middleware, wireless decentralized photo-based governance of devices is enabled. In a specific example, the photographic documentation created on the application at a primary care site would be transmitted and stored in a hospital information system to document the actions taken at a remote clinic.

According to a second aspect, there is provided an apparatus configured to host a data processing agent for processing data from one or more automated analyzers. The apparatus comprises a communications interface, a data memory, and a processor coupled to the communications interface and the data memory. The communications interface is configured to receive visual representation data of at least a display interface of an automated analyzer after the automated analyzer has performed a predefined operation. The visual representation data of the display interface comprises data associated with an outcome of the predefined operation performed by the analyzer. The processor is configured to process the visual representation data to extract data relating to an outcome of the predefined operation computed by the automated analyzer and comprised in the visual representation data associated with the predefined operation. The processor is configured to evaluate the data associated with the predefined operation according to at least one evaluation criterion to thus generate evaluation data. The processor is configured to store the evaluation data associated with the predefined operation.

According to a third aspect, there is provided a system for remote automated analyzer management comprising a computing device comprising a camera, at least one automated analyzer of biological samples, an apparatus configured to host a data processing agent for processing data from the at least one automated analyzers, and a communications network configured to communicably couple the computing device and the apparatus via a first communication channel, and the communications network is configured to communicably couple at least one automated analyzer and the apparatus via a second communication channel distinct from the first communication channel.

The computing device is configured to obtain, via the computing device, visual representation data of at least a display interface of an automated analyzer after the automated analyzer has performed a predefined operation, wherein the visual representation data of the display interface comprises data associated with an outcome of the predefined operation performed by the analyzer.

The computing device and/or the apparatus are configured to process the visual representation data to extract data relating to an outcome of the predefined operation computed by the automated analyzer and comprised in the visual representation data associated with the predefined operation.

The computing device and/or the apparatus are configured to evaluate the data associated with the predefined operation according to at least one evaluation criterion to thus generate evaluation data.

The computing device and/or the apparatus are configured to store the evaluation data associated with the predefined operation.

According to a fourth aspect, there is provided a computer program element comprising machine readable instructions which, when executed by a processor, cause the processor to perform the computer implemented method according to the first aspect, or its embodiments.

Optional embodiments are defined in the dependent claims, to which the reader may now refer, and which are discussed further in this specification.

Certain terms will be used in this patent application, the formulation of which should not be interpreted to be limited by the specific term chosen, but as to relate to the general concept behind the specific term.

As used herein, the terms "comprises," "comprising," "includes", "including", "has", "having", or any other variation thereof, are intended to cover a non-exclusive inclusion.

The terms "patient sample" and "biological sample" refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis, or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semisolid biological material is rendered liquid by dissolving or suspending it with a suitable liquid medium. In some embodiments, the sample is suspected to contain a certain antigen or nucleic acid.

The term "automated analyzer" as used herein encompasses any apparatus for obtaining measurement values relating to a medical condition of a patient.

In one example, the automated analyzer may be an automated analyzer of medical samples for obtaining a measurement value relating to a medical condition of a patient. For example, an automated analyzer may measure light absorption, fluorescence, electrical potential or other physical or chemical characteristics of the reaction to provide the measurement value. Often such patient samples are treated before analytical testing is done. Blood sampled from a patient is e.g. centrifuged to obtain serum or treated with anti-coagulants to obtain plasma.

Analytical testing by an analyzer has the goal of determining the presence and/or concentration of an analyte in a patient sample. The term "analyte" is a general term for substances for which information about presence and/or concentration is intended. Examples of analytes are e.g. glucose, coagulation parameters, endogenic proteins (e.g. proteins released from the heart muscle), metabolites, or nucleic acids.

An "automated analyzer" may comprise a portable appliance that can be communicatively connected to a smartphone, tablet PC, or other computing device via a USB (TM), WiFi (TM), or Bluetooth (TM) connection, for example. Such a portable appliance may be configured to perform analytical testing.

An "automated analyzer" may be configured to be usable in the vicinity of a patient ward, in which case it is often referred to as a "Point of Care (POC) device." However, the techniques discussed herein are not limited to POC devices and may be applied to many types of laboratory analysis systems that generate message data.

The term "Point of Care" POC or "Point of Care environment" as used herein is defined to mean a location on or near a site of patient care where medical or medically related services such as medical testing and/or treatment are provided, including but not limited to hospitals, emergency departments, intensive care units, primary care setting, medical centers, patient homes, a physician's office, a pharmacy, or a site of an emergency.

The term "point of care testing" POCT as used herein encompasses analysis of one or more patient sample(s) in a point of care environment. POCT is often accomplished through the use of transportable, portable, and handheld instruments, but small bench automated analyzers or fixed automated analyzers can also be used when a handheld device is not available- the goal being to collect the patient sample and obtain the analytical data in a (relatively) short period of time at or (relatively) near the location of the patient.

POCT is performed using various POC automated analyzers such as (but not limited to) analyzers for glucose, coagulation, blood gas, urinalysis, cardiac and molecular testing. Results may be viewed directly on the POC analyzer(s) or may be sent to the POCT system and displayed in a Laboratory Information System with central lab results, or alongside imaging results in a Hospital Information System.

The term "point of care device management system" (POC-DMS) as used herein denotes a data processor configured to communicate with, and manage, one or more POC devices via a computer network to enable a POC coordinator to manage the POC devices, or to enable maintenance personnel to monitor the equipment. Optionally, the POC-DMS is a terminal computer connected to the same network that the POC devices are connected to. Optionally, the POC-DMS may be provided as a server, virtual machine or a virtualized server hosted remotely to the network that the POC devices are connected to, enabling remote management of the POC devices. It is not essential that the POC devices (automated analyzers) are connected to the same subnet, or network branch, for example, as the POC-DMS.

The term "portable computing device" encompasses any electronic appliance that can be moved easily from one location to another, in particular any handheld battery powered mobile appliance, including but not limited to a cellular telephone, a satellite telephone, a pager, a personal digital assistant ("PDA"), a smartphone, a navigation device, a smart book or reader, a combination of the aforementioned devices, a tablet computer or a laptop computer.

The term "patient health parameter" as used herein encompasses any aspect of a patient's physiology that is measurable or indicated by an analysis of a patient sample for one or more analytes. Therefore, an automated analyzer may be used in a point of care environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Results may be viewed directly on the POC analyzer(s) or may be sent to the POCT system and displayed in a Laboratory Information System with central lab results, or alongside imaging results in a Hospital Information System.

In the field of bedside testing or point of care testing, the testing is typically performed by nurses, medical staff, or doctors but also pharmacists who are collectively called "operator(s)" or "users" herein. However, anyone who possesses the required certification may be an operator. A point of care coordinator POCC may be at the same time an operator of POC analyzer(s) and an operator of POC analyzer(s) may be at the same time a point of care coordinator POCC and thus user of portable computing device(s).

The term "analytical data" as used herein encompasses any data that is descriptive of a result of a measurement of one or more patient health parameter(s) performed by a POC analyzer of the biological sample that has been analyzed. In the case of a calibration the analytical data comprises the calibration result, i.e., calibration data. In particular, the analytical data comprises an identifier of the patient sample for which the analysis has been performed and data being descriptive of a result of the analysis, such as measurement data.

The term "communication side channel" refers to an alternative means of communication between a legacy automated analyzer and a computing apparatus intended to obtain data from the legacy automated analyzer.

The use of a communication side channel does not mean that a legacy communication channel must also be present. For example, the photograph or video-based side channel discussed in the present specification can be used to communicate information from the GUI display of a legacy automated analyzer that has no communication interface of its own. Alternatively, the photograph or video-based side channel discussed in the present specification can be used to communicate information from the GUI display of a legacy automated analyzer that has a disconnected legacy communications interface. In embodiments, the legacy automated analyzer may still communicate with, for example, a POC-DMS according to a legacy communication scheme. In this case, a data processing agent hosted on an apparatus can signal additional information to the POC-DMS that has been computed based on visual representation data sent to the apparatus comprising the data processing agent via a side channel.

The communication side channel is, in an example, established by a device intermediate to the first and second computing apparatuses. For example, a legacy automated analyzer communicates information such as status data, result data, and the like according to a protocol that is fixed and difficult to alter in the legacy automated analyzer without an embedded software upgrade. For example, the legacy automated analyzer may communicate information to a computing apparatus according to the "HL7" or health level 7 protocol developed by the "HL7" international standardization organization, or one of the precursor standards to HL7. Alternatively, the legacy automated analyzer may communicate with the computing apparatus via a protocol such as Ethernet, or a proprietary protocol operating over Ethernet.

Therefore, the "communication side channel" is established between the legacy automated analyzer and the computing apparatus, for example, by obtaining a photograph and/or video of a display of the legacy automated analyzer, and then transmitting the photograph and/or video over, for example, a WiFi (TM), Bluetooth (TM), 3GPP (TM) communication link to an apparatus comprising a POC-DMS. The photograph and/or video of the display of the legacy automated analyzer is visual representation data. The photograph and/or video of the display of the legacy automated analyzer can be processed using image processing techniques, and an evaluation criterion applied to the processed visual representation data. In this way, extra data may be extracted from the display of the legacy automated analyzer that is not comprised in the communication schema of the legacy automated analyzer. The use of the communication side channel also enables extra certification, location, or authentication data to be appended, or logically linked, to the visual representation data transmitted over the communication side channel. The extra data appended, or logically linked, to the visual representation data is obtainable, for example, from an application environment of the computing operators used to obtain the visual representation data of the legacy automated analyzer. Legacy automated analyzers can be integrated into modem point of care data management systems (POC-DMS).

The term "computing device comprising a camera" encompasses, for example, a smartphone (for example, an Apple iPhone (TM) or a Google Android (TM)-based smartphone. Alternatively, a "computing device comprising a camera" can also comprise a tablet such as an Apple "iPad" "computing device comprising a camera" or a Windows "Surface." The computing device comprises, for example, a touch screen display on a front side, and a camera on the rear side. Therefore, a user of the computing device can obtain visual representation data of a display screen of a legacy automated analyzer by photographing or videoing the graphical user interface displayed by the legacy automated analyzer at a predetermined stage of a predetermined process carried out by the legacy automated analyzer. The term "computing device comprising a camera" also encompasses, for example, a personal computer or a laptop comprising an integrated web camera, because an integrated web camera of such a computer can be positioned such that a display screen of a legacy automated analyzer is within the field of view of the integrated web camera. The term "computing device comprising a camera" also encompasses, for example, a personal computer or a laptop that is communicably coupled to an external web camera, when the external web camera has a field of view encompassing the display screen of a legacy automated analyzer. The term "computing device comprising a camera" may also encompass an intelligent video camera equipped with a communications interface and capable of performing image processing according to the aspects discussed herein.

The term "visual representation data" encompasses image data formatted as .APNG, .PNG, .AVIF, .GIF, .JPEG, .SVG, .BMP data, and the like. If the "visual representation data" is a video, it can be in one of the .MP4, .MOV, .WMV, .FLAC, .AVI, .FLV format, and the like. In other words, the "visual representation data" is obtained in formats readily obtainable from a typical smartphone or smart tablet comprising a camera. The "visual representation data" is, thus, able to capture substantially all details of information displayed on the display interface of an automated analyzer. In some cases, the "visual representation data" is divided into a representation of the display of the automated analyzer, and a representation of the surrounding area of the display of the automated analyzer. For example, labels, barcodes, and/or identifiers added to the front of a point of care device using permanent marker pen, for example, may enable unique identification of a legacy automated analyzer at a remote data processing agent. In some improvements, capturing such artefacts in the vicinity of a display screen on the casing of a legacy automated analyzer may improve analyzer and/or user authentication.

The term "predefined operation" as performed by an automated analyzer encompasses substantially any action that can be performed by a legacy automated analyzer, to the extent that a sign that the predefined operation has been performed can be derived from the display interface of the legacy automated analyzer, or in the vicinity of the legacy automated analyzer, using video or image analysis. For example, the completion of a specific type of test is an example of a "pre-defined operation," and can be identified by image recognition algorithms applied to the visual representation data obtained from the display of the legacy automated analyzer. In many cases, legacy automated analyzers perform "predefined operations" such as operator certification, quality control, and operator training functionality using hard- coded menus based on the analyzer itself. Certification, quality control, and training results are not, in many cases, signalled to a centralised POC-DMS, and signalling for transferring such information is often not included in the protocols of many legacy automated analyzers.

The term "location data" is typically obtained by a location service of the computing device comprising a camera. For example, the "Core Location" service of the Apple iPhone (TM) or iPad (TM) can resolve device location based on one or a combination of GPS (global positioning system), magnetometer readings, accelerometer readings, and network-based location services from mobile operators or wireless access point operators, for example. A processor of a computing device can interrogate the location service. The location service returns, for example, a grid reference (for example, with a given degree of uncertainty). This location data can be compared to a database of known locations of automated analyzers, where the locations of the automated analyzers are stored in a POC-DMS, for example. Accordingly, the location data enables the location of the computing device comprising a camera to be localized relative to one or more legacy automated analyzers. One use of such information is that a data processing agent can verify the authenticity of a user who has sent a result in the form of visual representation data to a data processing agent, where the visual representation data has been captured from a graphical user interface of a legacy analyzer. The data processing agent can compare the location data obtained from the location service of the computer comprising a camera with the known location of a legacy analyzer. The data processing agent can apply a geofence around a legacy analyzer, so that visual representation data sent by a user from outside the geofence around the legacy analyzer is not considered valid.

The term "logical link" encompasses a method enabling a first record, or item of data, to identify at least a second record, or item of data, during a lookup or search process. For example, a logical link may be a key value of a database.

The term "reconfiguration command" encompasses instructions sent in the form of data from, for example, a data processing agent and/or a POC-DMS to at least one automated analyzer of an analyzer system. Automated analyzers often have the capability to be addressed according to a communication schema, so that adjustments to an automated analyzer can be made remotely. According to an embodiment, reconfiguration command can disable or lock a legacy automated analyzer, optionally displaying a relevant message to any user of the automated analyzer. According to an embodiment, a user of the automated analyzer with user level privileges may unlock the automated analyzer. According to an embodiment, the reconfiguration command is to lock, or to freeze, the automated analyzer until a higher authority operator such as a supervisor unlocks the automated analyzer using, for example, a POC-DMS.

According to an embodiment, a reconfiguration command can cause a message to be displayed to a user on the screen of a legacy automated analyzer. For example, a data processing agent and/or a POC-DMS may report to a user, via a user interface of a legacy device, that a submitted photographic or video image comprising visual representation data has not been accepted by the data processing agent, and that the image should be taken again, for example.

According to embodiments, the reconfiguration command can configure one, or more, of the following parameters of an automated analyzer: screen formatting settings, language settings, date or time format settings, shutdown, sleep, hibernate, logout, or timeout settings, connection configuration, analyzer status, security parameters, words, authentication mechanism, login mechanism (such as specifying only a user identifier, user identifier plus password, or a user identifier plus barcode). The reconfiguration command may further configure patient identification or mapping parameters, a patient identification mechanism, default measurement units, quality control parameters, quality control lockout functions, physical or logical location of the analyzer, and location specific authentication and authorization data.

The term "communication network" as used herein encompasses any type of wired or wireless network, including but not limited to a WIFI, GSM, UMTS or other wireless digital network or a wired network, such as Ethernet or the like. For example, the communication network may include a combination of wired and wireless networks. Automated analyzer status data may be transmitted over the communication network.

The term "server" encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine (JVM), or even to separate virtual machines running different Operating Systems on the same physical machine and sharing that machine's computing resources. Servers can run on any computer including dedicated computers, which individually are also often referred to as "the server" or shared resources such as virtual servers. In many cases, a computer can provide several services and have several servers running. Therefore, the term server shall encompass any computerized device that shares a resource with one or more client processes. The server can receive, process, and transmit automated analyzer status data.

The term "server interface" encompasses any hardware-, firmware- and/or software- based module operable to execute program logic to allow communication with an external entity (such as a server or another interface).

The term "data processing agent" refers to a computer implemented software module executing on one or more computing devices, such as a server, that is able to receive automated analyzer status data from a point of care device, and annotation data from a user or operator, and associate the automated analyzer status data and the annotation data. The "data processing agent" may be implemented on a single server, or multiple servers, and/or an internet-based "cloud" processing service such as Amazon AWS (TM) or Microsoft Azure (TM). The "data processing agent," or a portion of it, may be hosted on a virtual machine. The data processing agent can receive, process, and transmit automated analyzer status data.

The term "user interface" encompasses any suitable piece of software and/or hardware for interactions between an operator and a machine, including but not limited to a graphical user interface for receiving as input a command from an operator and to provide feedback and convey information thereto. Also, a system / device may expose several user interfaces to serve different kinds of users/ operators. The user interface may display items of automated analyzer status data.

### Description of the Drawings

- **FIG.1**: schematically illustrates a system according to the third aspect.
- **FIG.2**: schematically illustrates a computer implemented method according to the first aspect.
- **FIG.3**: schematically illustrates an example of an automated analyzer and a test cartridge.
- **FIG.4**: schematically illustrates an example of visual representation data obtained from a display of an automated analyzer by a camera.
- **FIG.5**: schematically illustrates extracting information from the visual representation data.
- **FIG.6**: schematically illustrates an example of a process of a complete user interaction.
- **FIG.7**: schematically illustrates examples of data structures composed from the processed visual representation data and user data.
- **FIGs.8A-C**: schematically illustrate three exemplary graphical user interface screens for guided result documentation using a computer comprising a camera.
- **FIGs.9A-C**: schematically illustrate three exemplary graphical user interface screens for guided remote device registration and quality control testing.
- **FIG.10A**: schematically illustrates a graphical user interface screen for guided remote quality control testing.
- **FIG.10B**: schematically illustrates another graphical user interface screen for guided remote quality control testing.
- **FIG.11A**: schematically illustrates a graphical user interface for user management.
- **FIG.11B**: schematically illustrates another graphical user interface for user management.
- **FIG.12**: schematically illustrates a computer comprising a camera.
- **FIG.13**: schematically illustrates an apparatus according to the second aspect.
- **FIG.14**: schematically illustrates communication in a system according to the third aspect.

Note: The figures are not drawn to scale, are provided as illustration only and serve only for better understanding but not for defining the scope of the invention. No limitations of any features of the invention should be inferred from these figures.

### Detailed Description

Point of Care (POC) analyzers (also known as automated analyzers of medical samples) are commonly managed by a server, and in particular, a hardware management server, also called Point of Care Data Management System (POC-DMS). Such a server provides connectivity for POC analyzers and management of test results, operators, quality controls, and analyzers. For example, one POC-DMS could manage all POC analyzers in a hospital, hospital department, or medical testing center.

Management of POC systems is challenging - there can be dozens of sites, hundreds of POCT devices/kits, and thousands of operators to manage to assure quality of testing. Furthermore, point of care testing in primary care is extremely heterogeneous with many different point of care analyzers from very basic ones to powerful diagnostic instruments. Furthermore, some point of care analyzers are legacy analyzers which are difficult to integrate into a point of care data management system. Independent general practitioners and small clinics may have between one and ten point of care analyzers. There may be a varying level of training between members of staff, and varying sophistication of internal processes of such small clinics.

In some cases, independent general practitioners and healthcare laboratories may own a single point of care analyzer for measuring one parameter. Specific quality management activities on such instruments may be performed rarely. When compliance and quality management activities are documented with manual "pen and paper" techniques, integrating results into the healthcare ecosystem is costly and requires either additional hardware, or manual to electronic data transcription, which may itself introduce error and confidentiality problems.

The POC team should usually hold the responsibility for determining the test menu, selecting technologies, establishing policies and procedures, ensuring training and regulatory compliance, and providing advisory assistance to the end operators of POC technologies.

In general, to address the above-mentioned concerns, image- based governance of distributed automated analyzers is proposed in this specification. In one general example, a member of a POC team can use a smartphone equipped with a camera and a smartphone application to perform a guided instrument workflow, to obtain photographic documentation of results of the actions directly from the legacy point of care analyzer, and to analyse the photographic documentation to generate data that can be input into a POC-DMS in a secure and organised manner. The data obtained from the photographic documentation is transmitted to the POC-DMS via a side channel, and this there is no requirement to generate specific computer code to connect a legacy automated analyzer to a more modem POC-DMS.

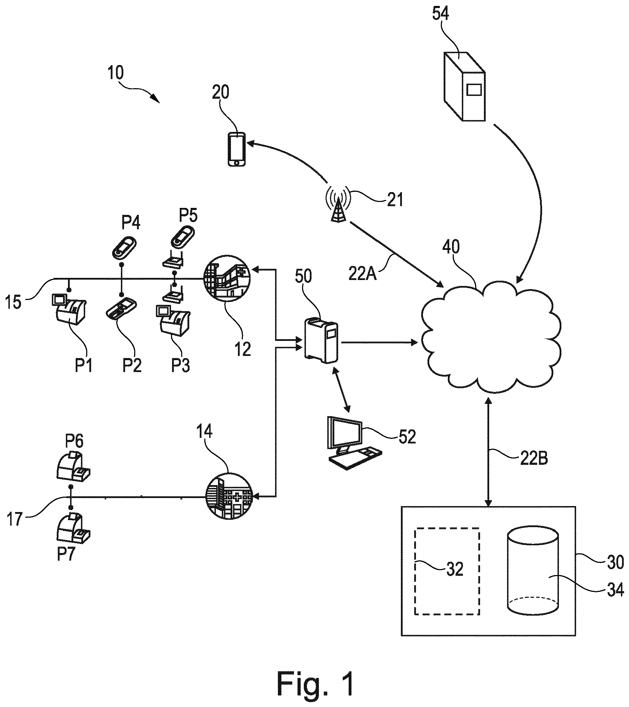

FIG.1 schematically illustrates a system according to the third aspect.

The system 10 may comprise one, or more, local area networks (LANS) or wide area networks (WANS). For example, the first clinic 12 comprises a first local area network 15. A second clinic 14 comprises a second local area network 14. The first local area network 15 is communicably coupled to a plurality of automated analyzers P1-P5. For example, the automated analyzer P1 may be a modem Cobas (TM) Liat (TM) device. Other automated analyzers in the first local area network 15 can be provided by the same or other manufacturers, and in some cases may be legacy automated analyzers. The second local area network 17 comprises automated analyzers P6 and P7. Therefore, the first local area network 15 is indicative of a local area network installed in a hospital requiring a large diagnostic suite. The second local area network is indicative of a local area network installed in a small clinic, for example.

The system 10 further comprises a point of care device management system (POC-DMS) 50. The POC-DMS 50 may be operated, for example, by a healthcare provider to enable oversight of a number of automated analyzers in a healthcare system. A POC-DMS 50 is a hardware management server for providing connectivity for automated analyzers P1-P7 and management of test results, operators, quality controls, and analyzers. POC-DMS 50 accessed, for example, by a local access computer 52 or, for example, via remote access using Remote Desktop (TM) or SSH, for example.

The POC-DMS 50 forms an interface between, for example, one or more internal hospital LANs 15, 17 and a WAN such as the internet, or a larger healthcare organization network distributed over many locations, using an active directory system, for example.

For example, the POC-DMS 50 may comprise a communications adaptor capable of communicating with automated analyzers P1-P7 using the health level 7 (HL7) protocol. Other automated analyzers may be configured to communicate via Ethernet, WiFi (TM), and/ or Bluetooth (TM), for example. Therefore, the POC-DMS 50 is, in embodiments, provided with Ethernet, WiFi (TM), and/ or Bluetooth communications interface.

In the field of point-of-care testing, testing can be performed by nurses, medical staff, or doctors who can be collectively referred to as "users" or "operators" of a POC analyzer. Users of an automated analyzer typically initial certification, and periodic recertification. A Point of Care Coordinator (POCC) may be an operator but also a manager responsible for administering initial certifications, and recertifications. For example, the POCC may be able to access special administrative privileges on the POC-DMS, whereas the standard automated analyzer users can only log onto the automated analyzers of the first and/or second networks.

The term "certification" can encompass many forms of confirmation of certain characteristics (such as training, examination, or educational background or accreditation) of an operator. A certification can denote an entry of a user on a list of certified operators of one or more types of automated analyzer comprised in, for example, the first network 15. The certification may be permanent or time restricted. In an embodiment, certification can be performed using an automated analyzer P1 of, for example, the first network 15.

The POC-DMS 50 is communicably coupled to a communications network 40, such as a wide area network, for example the internet. In some embodiments, the POC-DMS 50 is communicably coupled to other communication nodes in a system using a virtual private network or active directory system (not shown).

The system 10 further comprises a computer 20 such as a smart phone or smart tablet. The smart phone or smart tablet is communicably coupled to the communications network 40 via, for example, a WiFi access point or 3GPP base station. The smart phone or smart tablet is configured to operate an application for remote automated analyzer monitoring, as will be subsequently described.

System 10 further comprises apparatus 30 that is communicably coupled to the POC-DMS 50 via the communications network 40. The apparatus 30 hosts a data memory 34 and a data processing agent 32. According to embodiments to be discussed subsequently, the data processing agent 32 is configured to receive visual representation data 70 from the computing device 20 comprising a camera of at least one automated analyzer comprised in the first 15 and/or second local area networks 17. In other words, the computing device 20 can communicate visual representation data 70 of at least one of the automated analyzers P1-P7, to the data processing agent 32 hosted by the apparatus 30 via communication links 22A and 22B without needing to communicate via the POC-DMS 50. In the words, the computing device 20 can communicate visual representation data of at least one of the automated analyzers P1-P7 to the apparatus 30 via a side-channel.

According to an embodiment, computing device 20 performs image processing on the visual representation data to extract data relating to an outcome of a predefined operation. In this embodiment, computing device 20 communicates the data associated with the predefined operation to the apparatus 30 via the side channel, rather than the visual representation data 70.

According to an embodiment, apparatus 30 is configured to communicate evaluation data associated with a predefined operation to the POC-DMS 50.

According to an embodiment, apparatus 30 is configured to communicate a reconfiguration command of the POC-DMS 50 from the apparatus 30 to the POC-DMS 50 dependent on evaluation data associated with a predefined operation.

According to an embodiment, apparatus 30 is configured to communicate a reconfiguration command of an automated analyzer P 1-P7 to a respective automated analyzer evaluation data associated with a predefined operation.

Beneficially, aspects of remote automated analyzer monitoring can therefore be performed by obtaining visual representation data 70 via a display interface of an automated analyzer, and/or via a user operated device such as a smart phone or smart tablet.

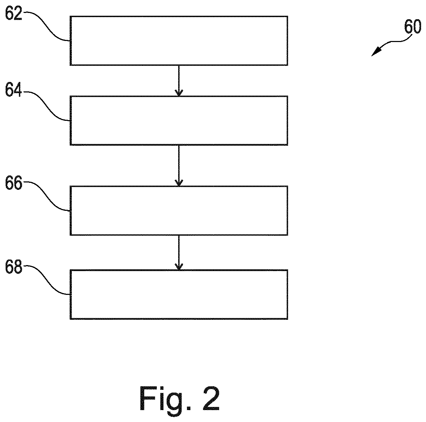

**FIG.2** schematically illustrates a computer implemented method according to the first aspect.

According to a first aspect, there is provided a computer implemented method 60 for remote analyzer monitoring. The method comprises:
- obtaining 62, via a computing device 20 comprising a camera 21, visual representation data 70 of at least a display interface P1-D of an automated analyzer P1 after the automated analyzer P1 has performed a predefined operation, wherein the visual representation data 70 of the display interface P1-D comprises data associated with an outcome of the predefined operation performed by the automated analyzer P1;
- processing 64 the visual representation data to extract data 74 relating to an outcome of the predefined operation computed by the automated analyzer P1 and comprised in the visual representation data 70 associated with the predefined operation;
- evaluating 66 the data associated with the predefined operation according to at least one evaluation criterion to thus generate evaluation data 76; and
- storing 68 the evaluation data associated with the predefined operation.

According to an embodiment, the remote analyzer monitoring is performed via a communication side channel 22A, 22B.

According to an embodiment, the evaluation data associated with the predefined operation is displayed on a user interface of a user device.

According to the first aspect, a method of photo-based governance of distributed point-of-care devices is provided that is easy to deploy within healthcare organizations. No reverse engineering of a legacy automated analyzer, or provision of additional interface electronics, is required.

A POC user can use their phone. According to one embodiment, a standard camera application of a smartphone operating environment can be used to capture visual representation data 70 of the automated analyzer, and a standard email or messaging client can be used to transmit the visual representation data 70 to a corresponding web or messaging server hosted by the data processing agent 32 of the apparatus 30. The advantage of this is that no special smartphone software is needed to transmit visual representation data 70 to the data processing agent 32.

According to another embodiment, computer 20 comprising a camera 21 operates an application that appends user-relevant metadata to visual representation data 70 prior to transmission to the data processing agent. For example, the application may append a user identifier of the POC device to the visual representation data 70 . Furthermore, the provided smart phone application, for example, can be configured to present an illustrated guided instrument workflow to a user, and to provide photographic documentation of results, QC tests, or consumable use. A given POC user will be presented with a list of automated analyzers connected to a local area network 17 in a healthcare facility where the POC user is registered as an operator.

The step of obtaining 62 visual representation data 70 of display interface P1-D involves directing a field of view of a camera 21 of a computing device 20 so that it can capture at least a display interface P1-D of an automated analyzer P1. The capabilities of smartphone cameras mean that many types of legacy display interfaces can be imaged. For example, colour LCD or OLED displays, touchscreens, as well as black-and-white matrix displays capable of displaying 2D images and text can be imaged. However, simpler displays such as seven-segment liquid crystal displays for displaying numerical results can be imaged and captured as visual representation data 70.

According to an embodiment, the visual representation data 70 comprises image data of at least the display interface of the automated analyzer P1-D. Optionally, the visual representation data 70 also comprises metadata added to the image data by the camera 21 of the computing device 20. For example, the metadata comprises a date, time, and location at which the visual representation data 70 was obtained. According to an embodiment, the metadata comprises user identification information, or login information, of the application software executed by the computing device 20 so that the identity of a user obtaining the visual representation data 70 can be stored.

The field of view of the camera 21 can also capture peripheral regions of the area surrounding the display interface P1-D of the automated analyzer P1. In an embodiment, camera 21 is configured to capture an image of the entire automated analyzer P1, comprising the display interface of the automated analyzer.

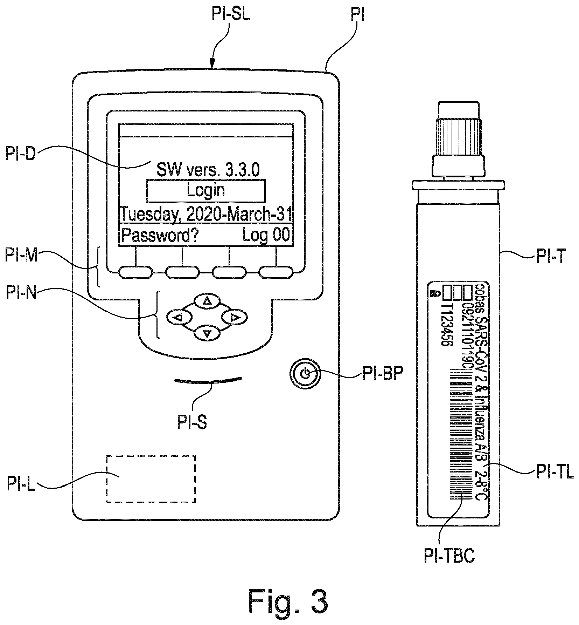

**FIG.3** schematically illustrates an example of an automated analyzer and a test cartridge.

The automated analyzer P1 illustrated in **FIG. 3** uses disposable cartridges to perform a predefined operation such as testing of patient samples, certification, and analyzer quality control checks. A test cartridge P1-T may comprise an identifying label P1-TL and specifically an identifying barcode P1-TBC. In use, the test cartridge can firstly be identified by the automated analyzer P1 when a user registers the test cartridge P1-T via a barcode reader P1-S of the automated analyzer. The user then inserts the test cartridge P1-T into a testing slot P1-SL of the automated analyzer, in this case located on the lid of the automated analyzer.

The automated analyzer comprises a display screen P1-D here illustrated at a login step of the automated analyzer P1. The interface of the automated analyzer P1 comprises a row of four menu interface buttons P1-M, and a cluster of four navigation interface buttons P1-N. A general power switch P1-BP is provided on a portion of the front of the automated analyzer P1. A barcode scanner P1-S is provided underneath the cluster of navigation buttons. For example, the barcode scanner P1-S can be utilized in a user logon process, and/or to identify test cartridges for insertion into the automated analyzer P1.

The display screen P1-D will accompany a typical patient sample test, certification test, or analyzer quality control check using a plurality of user feedback masks displayed on the display screen P1-D. At least one of the user feedback masks will comprise a result of the predefined operation such as a patient sample test, certification test, or analyzer quality control check. Therefore, visual representation data 70 of the display screen P1-D obtains a likeness of user feedback mask displayed by the display screen P1-D. Additionally, the camera 21 can be used to capture a likeness of the test cartridge P1-T as supplementary to the visual representation data. In an embodiment, camera 21 can be used to capture a likeness of the test cartridge P1-T during insertion into the testing slot P1-SL, to verify that the correct test cartridge has been inserted into the automated analyzer P1. In an embodiment, a region of the casing of the automated analyzer comprises an identifying label P1-L of the automated analyzer. Such identifying labels P1-L can also form part of the visual representation data 70.

In an embodiment, the visual representation data 70 comprises a first region 100 comprising a likeness of the display interface P1-D. In an embodiment, the visual representation data 70 comprises a second region 102 comprising a likeness of the peripheral regions of the area surrounding the display interface P1-D. In an embodiment, the second region 102 comprises at least one likeness of a manufacturer logo. In an embodiment, the second region 102 comprises at least one likeness of a label comprising an identifier code of an owner of the automated analyzer P1. Unique labels P1-L and codes comprised on the body of the automated analyzer P1 may, for example, be used to uniquely identify the automated analyzer P1.

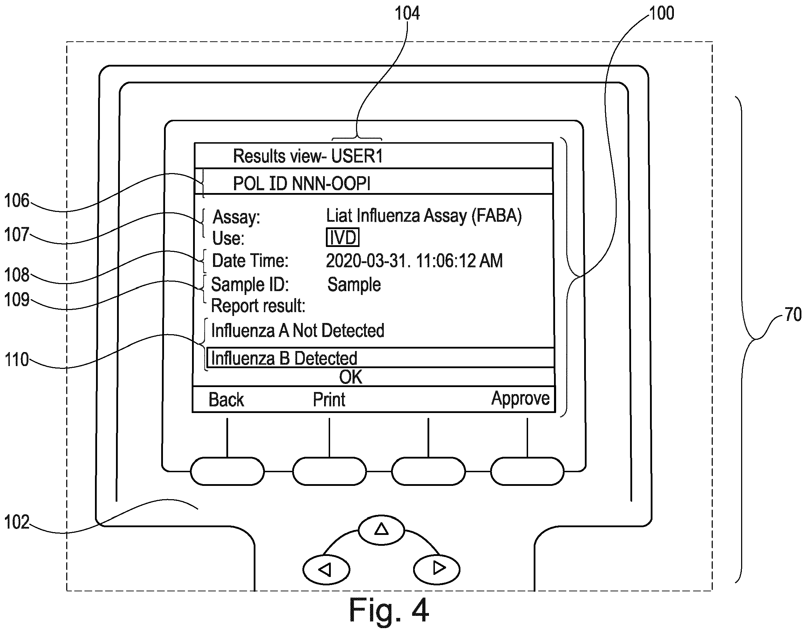

**FIG.4** schematically illustrates an example of visual representation data obtained from a result display mask of an automated analyzer by a camera.

According to an embodiment, the result display mask is displayed by an automated analyzer P1 at the conclusion of one or more predefined operations performed by the automated analyzer P1. The result display mask may provide, for example, a visual patient test result, a visual user certification result, a visual quality control result, a visual consumable inventory, a visual hardware status update, although other types of result display masks of one or more predefined operations can also be provided. Furthermore, the visual representation data 70 can comprise one or more intermediate display masks (not illustrated). Obtaining one or more intermediate display masks may be particularly useful during a user certification process, illustrating user compliance with a plurality of test steps during a certification process.

The visual representation data 70 obtained by the camera 21 is, for example, a two-dimensional image in one of a range of image formats known to a skilled person, such as JPEG. The visual representation data 70 provides a likeness of the display interface P1-D of an automated analyzer P1, and optionally a peripheral area 102 surrounding the display interface P1-D. According to an embodiment, the visual representation data 70 is processed to crop the peripheral area 102 surrounding the display interface P1-D, in cases where the peripheral area 102 contains no information useful for identifying the automated analyzer P1.

In an embodiment, the visual representation data 70 comprises a first screen region 104 containing a username of a user of the automated analyzer P1. The username of the user of the automated analyzer can be used, for example, to form a logical link between the visual representation data 70 and a database of users of a system 10 of automated analyzers P1-P7 curated, for example, by a POC-DMS 50.

In an embodiment, the visual representation data 70 comprises a second screen region 106 comprising an explicit statement of the identifier of the automated analyzer. The identifier of the automated analyzer can be used to generate a logical link between the visual representation data 70, and a specific record of a specific automated analyzer stored in a POC-DMS 50.

In an embodiment, the visual representation data 70 comprises a third screen region 107 comprising an identifier of a type of test result, a result, test cartridge and/or a test process being presented on the screen of the display interface P1-D at the time instant that the visual representation data 70 was captured. Therefore, the third screen region 107 comprises an identifier of a predefined operation performed by the automated analyzer P1. The identifier of the predefined operation performed by the automated analyzer P1 can be used to create a logical link to a database in a POC-DMS 50 of valid predefined operations.

In an embodiment, the visual representation data 70 comprises a fourth screen region 108. The fourth screen region 108 comprises date and/or time references relative to a start time and/or an end time of a predefined operation. This information is useful because a data processing agent 32 can extract the start time and/or an end time of a predefined operation from the visual representation data 70, and compared with the image capture time comprised in metadata of the visual representation data 70 added by the camera 21 at the time of capture of the visual representation data 70. In other words, a data processing agent 32 can use a similarity in date and time between the content of fourth screen region 108 and date and time metadata in the visual representation data 70 as one proxy indicating authenticity of the visual representation data 70.

In an embodiment, the visual representation data 70 comprises a fifth screen region 109 containing a sample identification name.

In an embodiment, the visual representation data 70 comprises a sixth screen region 110 comprising data relating to an outcome of the predefined operation computed by the automated analyzer P1. In the case of a patient test result, the outcome of the predefined operation may be a binary test result such as "Influenza A not detected." In the case of other types of patient test result, the outcome of the predefined operation may be a numerical reading within specified test ranges. The patient test result may be a graph or selection from a list.

If the predefined operation is a quality control process, the sixth screen region 110 comprises a binary pass or fail criterion of the quality control process. Alternatively, the sixth screen region 110 may comprise numerical data summarizing the success or failure of the quality control process. Alternatively, the sixth screen region 110 may comprise a code word, a QR code, or set of keywords for decoding at a data processing agent 32 for providing more detailed quality control feedback.

If the predefined operation is a user certification process, the sixth screen region 110 may comprise a binary pass or fail criterion of the user certification process. Alternatively, the sixth screen region 110 may comprise feedback on aspects of the user certification process. For example, the sixth screen region 110 may comprise a summary of the amount of time that a user has spent on each stage of the user certification process. The sixth screen region 110 may comprise a summary of test scores of challenge questions presented during the user certification process.

If the predefined operation is a consumable check of the automated analyzer, the sixth screen region 110 may summarize a number of remaining consumables, including those at risk of depletion.

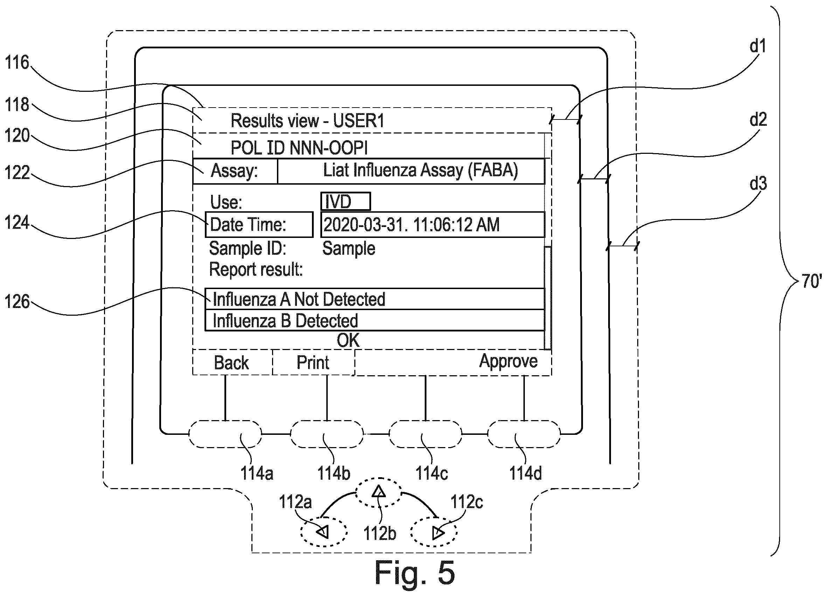

**FIG.5** schematically illustrates an example of extracting information from the visual representation data 70 of **FIG. 4****.**

A data processing agent 32 and/or a software application running on a computing device 20 comprising a camera 21 are configured to extract information from visual representation data 70 obtained at the conclusion, or during, a predefined operation carried out by the automated analyzer P1. Therefore, in one example the visual representation data 70 is transmitted to the data processing agent 32 (also comprising meta data associated with the visual representation data 70). Extraction of data relating to an outcome of a predefined operation is performed at the data processing agent 32. In another example, the visual representation data 70 is partially or fully processed at computing device 20 so that the data relating to the outcome of the predefined operation is transmitted to the data processing agent 32.

In an embodiment, an image processing algorithm and/or a trained model for image processing is applied to the visual representation data 70 to extract data from the visual representation data 70 relating to the outcome of the predefined operation.

The image processing algorithm may have access to a mask database comprising records of image processing masks for each possible automated analyzer P1-P7 present in the system 10. Furthermore, for each automated analyzer P1 comprised in the mask database, there may be a plurality of sub- records comprising image processing masks for all, or important, expected graphical user interface outputs that an automated analyzer P1 can generate, in use.

For example, image processing mask 116 contains alignment elements 112a-c and 114a-d corresponding to the menu selection buttons P1-M and navigation buttons P1-N, respectively. If the visual representation data 70 comprises artefacts resembling the menu selection buttons P1-M and navigation buttons P1-N, the image processing mask 116 can be more accurately registered to the visual representation data 70. In another example, unique features such as the menu selection buttons P1-M and navigation buttons P1-N appearing in the visual representation data 70 can be used to identify an image processing mask of a specific automated analyzer P1 in a data memory 34 of an apparatus 30 hosting the data processing agent 32. As another example, bevel features of a display screen can be identified (using edge detection algorithm, for example) and the relative separation distances of the bevels d1, d2, and d3 used to compute ratios that identify a specific automated analyzer P1. A skilled person will appreciate that a wide range of image recognition techniques can be used to automatically identify the automated analyzer P1.

According to another option, automated detection of the specific automated analyzer P1 is not necessary, and a user may use a menu of the application comprised on the computer 20 to select an automated analyzer.

Each image processing mask 116 defines fields corresponding to expected locations of data relating to the outcome of the predefined operation computed by the automated analyzer P1. For example, according to an embodiment, image processing mask 116 comprises a first region 118 in which a username is expected to be found. According to an embodiment, image processing mask 116 comprises a second region 120 in which an identification code of a specific automated analyzer is expected to be found. According to an embodiment, image processing mask 116 comprises a third region 122 in which a text string referring to a specific assay or test type is expected to be found. According to an embodiment, image processing mask 116 comprises a fourth region 124 comprising a date and/or time at which the outcome of the predefined operation is generated or displayed. According to an embodiment, image processing mask 116 comprises a fifth region 126 comprising an outcome of the predefined operation computed by the automated analyzer P1.

An image processing algorithm such as a text recognition algorithm is applied to one or more of the regions of the image processing mask 116 to thus extract the contents of the visual representation data associated with the predefined operation. According to an embodiment, a logical link with the meta data of the visual representation data 70 (where extant) is preserved.

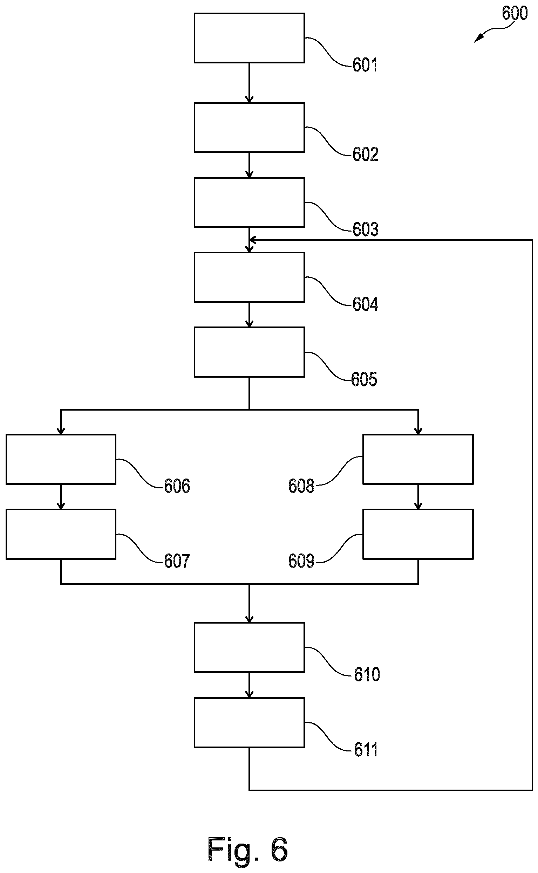

FIG.6 schematically illustrates an example of a process of a complete user interaction.

At exemplary step 601, a user loads an application on the computer 20 comprising a camera 21 intended to capture the visual representation data 70 of an automated analyzer P1. For example, the application could be available on the application store of an iPhone (TM) or Android (TM) smartphone, for example. When the application is loaded on the computer 20, the computer 20 initiates a logical connection between the application and, for example, a data processing agent 32 instantiated on a remote apparatus 30 and/or a POC DMS 50. In an embodiment where visual representation data 70 is sent from the computer 20 via a general-purpose email client, no special application is necessary, and this step may be omitted.

At exemplary step 602, the application on the computer 20 is logically linked to the data processing agent 32. The user may identify a clinic 12, 14, accessible to the POC-DMS. This enables the application of the computer 20 to populate the application with a "virtual clinic" denoting, for example, analyzers P1-P5 of clinic 12. In this way, the user can more quickly identify a specific legacy automated analyzer. In an embodiment where visual representation data 70 is sent from the computer 20 via a general-purpose email client, no special application is necessary, and this step may be omitted.

At exemplary step 603, user can optionally add a new type of automated analyzer to the "virtual clinic" using a wizard, drop-down menu, and the like in the application hosted by the computer 20. In an embodiment where visual representation data 70 is sent from the computer 20 via a general-purpose email client, no special application is necessary, and this step may be omitted.

At exemplary step 604, the user performs a predefined operation on at least one automated analyzer P1 of clinic 12 that is also comprised in the "virtual clinic". For example, the predefined operation is one or more of a patient test, a quality control test, a certification procedure, consumable check, or a hardware self-test of the automated analyzer P1.

At exemplary step 605, the user obtains a photograph and/or video of the automated analyzer P1, including the graphical user interface (display interface) of the automated analyzer P1 at the time when the automated analyzer P1 is displaying a result, or an intermediate stage of the predefined operation. At least one, or a plurality, of items of visual representation data 70 is generated reflecting the state of the display interface of the automated analyzer at a result stage, or a plurality of intermediate stages of the predefined operation. The image and/or video may be obtained by a general-purpose imaging application of the computer, or from within a customized application that is capable of appending user metadata to the visual representation data 70.

Image processing and analysis can be performed entirely on computer 20, entirely at the data processing agent 32, or a mixture of both.

At exemplary step 606, image processing of at least one set of visual representation data 70 (and optionally the accompanying meta data) is performed by computing device 20.

At exemplary step 607, data associated with the predefined operation that has been extracted from the visual representation data 70 is transmitted to the data processing agent 32, optionally comprising meta data of the visual representation data 70 and stored in the data memory 34. Optionally, the original visual representation data 70 is also transmitted to the data processing agent 32 and stored in the data memory 34 with a logical link to the data associated with the predefined operation received by the data processing agent 32.

Alternatively, at step 608, the visual representation data 70 (and any associated meta data) is transmitted to the data processing agent 32 and optionally stored in the data memory 34.

At exemplary step 609, image processing of at least one set of visual representation data 70 (and optionally the accompanying meta data) is performed by the data processing agent 32 and stored in data memory 34.

At exemplary step 610, the data processing agent 32 evaluates the processed visual representation data 70 based on predefined rules and/or previous results. For example, if the predefined operation comprises a patient test result, the data processing agent 32 may forward the patient test result to the POC-DMS 50 for storage. If the predefined operation comprises a user certification result, the data processing agent 30 may forward the user certification result to the POC-DMS 50 for storage. In an embodiment, the user certification result may be used to control user access to the automated analyzer P1 used to perform the user certification test captured in the visual representation data 70.

At exemplary step 611, the visual representation data 70, any annotations, and the evaluation computed in step 610 is stored either in the data memory 34 of the apparatus, and/or the POC-DMS 50.

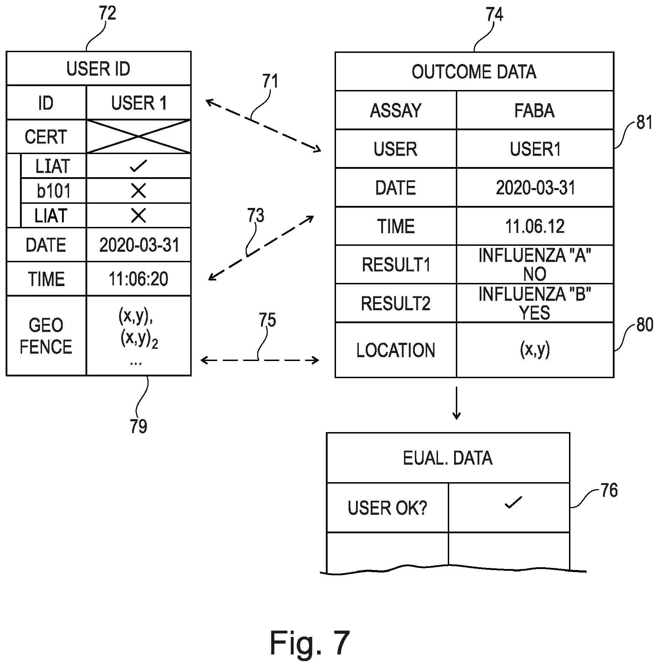

**FIG.7** schematically illustrates examples of data structures composed from the processed visual representation data and user data.

According to an embodiment, the computer implemented method 60 further comprises:
- obtaining, via a user interface of the computing device 20, an identifier 72 of a user performing the predefined operation using the automated analyzer P1; and
- generating at least one logical association between the identifier 72 of the user and the visual representation data 70 and/or evaluation data 74, wherein the at least one evaluation criterion is a comparison 71 of the identifier 72 of the user and a user record 81 of the data 74 relating to an outcome of the predefined operation.

For example, a user may enter an identifier 72 into a menu of an application hosted by the computing device 20. Alternatively, an identifier 72 of a user can be obtained by a data processing agent 32 in communication with the POC-DMS 50. In an embodiment, the user identifier 72 comprises a user identification code ID, and one or more certification fields defining types of automated analyzer that the user is certified to use. In an embodiment, the user identifier 72 comprises date and time fields defining the date and time that an application hosted by the computing device 20 observed a measurement being made. In an embodiment, the user identifier 72 comprises a location range (geo fence) 79. The location range or geo fence defines a range of permitted local or global locations within which a user identified by the user identifier 72 may use the certified automated analyzers.

As shown in **FIG.7****,** outcome data 74 is generated by step 64 of processing the visual representation data to extract data 74 relating to an outcome of the predefined operation computed by the automated analyzer P1.

In this exemplary embodiment, the outcome data comprises a record of the assay type, the user of the automated analyzer when the assay was taken, a date and time relating to a start or finish time when the assay was taken, assay results, and locations of the automated analyzer P1.

The data processing agent 32 is configured to generate a logical association 71 between a user record of the user identifier 72 and a user record 81 of the outcome data 74. In other words, the data processing agent 32 is configured to apply at least one evaluation criterion, in this case a match between the user identifier 72 and a user record 81 of the outcome data. If the user identifier 72 and the user record 81 of the outcome data do match, the data processing agent 32 can authenticate the fact that the user transmitting the visual representation data 70 is the same as the user who has logged in to the automated analyzer P1.

Accordingly, the step of evaluating the data 66 associated with the predefined operation according to at least one evaluation criterion may generate evaluation data 76 stating that the user of the automated analyzer P1 is a match to the user of the computing device 20, for the specific visual representation data 70 obtained by the computing device 20.

According to an embodiment, the evaluation criterion is a comparison of a first, second, and/or third time. The first time is a time at which the automated analyzer P1 reports to a POC-DMS 50 via a first (legacy) communication channel that a test has been performed. The second time is comprised in meta data of the visual representation data 70, wherein the second time is extracted from a date and time field 108 of the display of the automated analyzer P1. The third time is obtained from the operating environment of the computing device 20. Evaluating the first second and/or third times comprises ensuring that the first second and/or third times are close enough together relative to an absolute time measure. For example, a total time discrepancy of plus or minus five seconds, 10 seconds, or 20 seconds, 30 seconds, or 1 minute between the first, second, and/or third times may be tolerated, before the evaluation criterion states that the outcome of the predefined operation cannot be verified.

According to an embodiment, the computer implemented method further comprises:
- obtaining location data 79 of the automated analyzer P1 using a location service 25 of the computing device 20; and
- generating a logical association between the location data 79 of the automated analyzer P1 and the location data comprised in the evaluation data 80, wherein the at least one evaluation criterion is a comparison of location data 80 of the user and a user record 81 of the data 74 relating to an outcome of the predefined operation.

Also shown in **FIG.7****,** a logical link 75 can be defined between location data 80 comprised in meta data of the visual representation data 70 and the location range, or geo fence 79, of the user identifier 72. Therefore, an evaluation criterion evaluates whether, or not, the data 74 extracted from the visual representation data 70 was obtained from a location within the geofence permitted to a given user ID in the user identifier 72. If the data 74 extracted from visual representation data 70 was obtained from a location within the geofence, this is an additional indicator of trust authentication. In other words, it is indicated that the user was present next to the automated analyzer P1 at the time that the visual representation of data 70 was obtained.

Alternatively, if the data 74 extracted from the visual representation data 70 was not obtained from a location within the geofence, this may be an indicator for the data processing agent 32 to distrust, or to reject, the remainder of the data comprised in the outcome data 74. In such case of rejection, the evaluation data 76 may comprise a negative result. In response to a negative result, a command may be sent either to the computer 20, the automated analyzer P1, and/or the POC-DMS 50, of the reason for the negative result and or a demand to resend the visual representation data 70 from within the geofence. This improves authentication of visual representation data 70 obtained from an automated analyzer P1.

According to an embodiment, the computer implemented method 60 further comprises.
- detecting a predefined change of the display interface P1-D of the automated analyzer indicating that the automated analyzer P1 has completed the predefined operation; and
- upon detecting the predefined change of the display interface P1-D, automatically obtaining the visual representation data 70 using the camera 21 of the computing device 20.

The graphical user interfaces of automated analyzer P1 changing known ways, may be provided with known animations indicating that a predefined operation such as a result taking, certification, or the like are to be imminently reported. Accordingly, a predefined change of the display interface P1-D can be monitored using computing device 20. Camera 21 of computing device 20 is used in a monitoring (or "live") mode to monitor a video stream from the display interface P1-D. As one example, a "preparing result" dialogue with a clock icon is recognized by the computing device 20 is a prompt that an automated analyzer P1 is about to finish a predefined operation. After the completion of the predefined operation, visual representation data 70 is obtained using camera 21 of the computing device 20.

According to an embodiment, the computer implemented method further comprises:
- generating an identifier associated with the predefined operation to be performed on the automated analyzer P1 as defined in the at least one graphical instruction; and
- after obtaining the visual representation data 70 comprising the representation of the display interface P1-D of the automated analyzer P1:
   generating a logical association between the identifier and the visual representation data 70, and/or the stored evaluation data associated with the predefined operation; and
   storing the identifier.

According to an embodiment, the computer implemented method 60 further comprises:
- transmitting a reconfiguration command to automated analyzer P1 according to the evaluation data associated with the predefined operation; and
- reconfiguring the automated analyzer P1 based on the reconfiguration command, wherein the reconfiguration command is optionally a software lock of the automated analyzer P1, or a requirement for a user for perform a quality control or certification workflow.

Some automated analyzers P1-P7 may enable a bidirectional control link to the POC-DMS 50 and/or the data processing agent 32. For example, the communication stack of the automated analyzers P1-P7 may comprise legacy input commands enabling external devices to assume a degree of control over the respective automated analyzers P1-P7. In a specific example, the evaluation step 66 may conclude that the user of the computing device 20 is not certified to use a specific type of automated analyzer from which the outcome data 74 is generated. In this case, the reconfiguration command to the automated analyzer P1 may lock the automated analyzer P1 or prompt the user to perform a quality control or certification workflow.

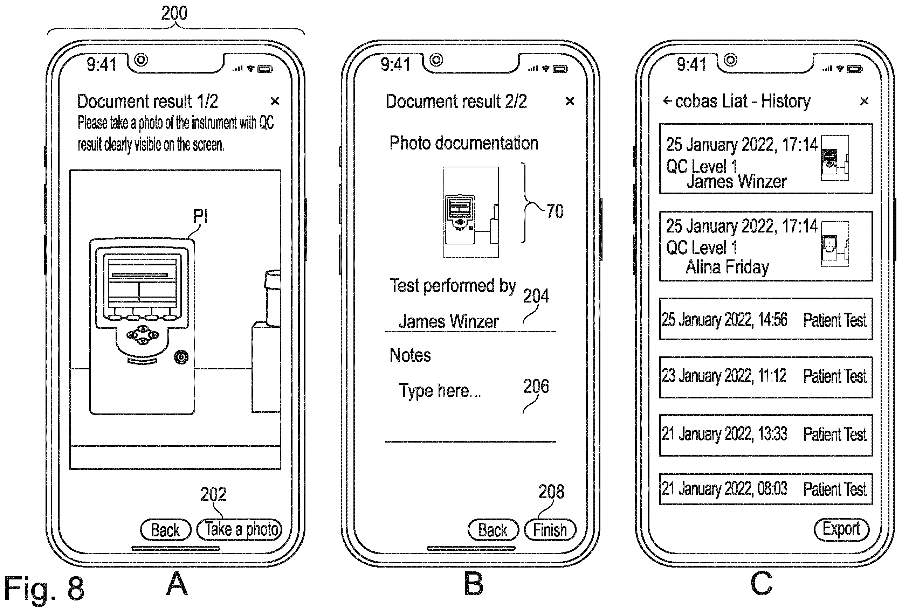

**FIG.8** schematically illustrates three exemplary graphical user interface screens for guided result documentation using a computer comprising a camera.

According to an embodiment, the computer implemented method 60 according to one of the preceding claims, further comprises:
- displaying, on a user interface 23 of the computing device 20, at least one graphical instruction associated with the predefined operation to be performed on the automated analyzer P1; and
- receiving, via the user interface of the computing device 20, a confirmation that the user intends to obtain the visual representation data 70, wherein the visual representation data 70 comprises a representation of a display interface of the automated analyzer when the automated analyzer has previously performed a predefined operation according to the at least one graphical instruction associated with the predefined operation.

**FIG. 8A****,** a first documentation result window 200 of a graphical user interface suitable for display on, for example, a display of the computing device 20 may prompt a user to take a photograph of the analyzer P1 with the results clearly visible on the screen of the analyzer P1. GUI button 202 is functionally coupled to a camera function of the computing device 20, and when the user actuates the GUI button 202 (for example, using a touchscreen), visual representation data 70 comprising, for example, a quality control test result is captured by the camera 21 of the computing device 20.

In **FIG. 8B****,** the user may add various annotations to the visual representation data 70 using the graphical user interface of the computing device 20. For example, a selection dialogue 204 enables a user to select their user identification 204 so that this is appended to the visual representation data 70. Furthermore, optional notes field 206 of the graphical user interface enables a user of the computing device 20 to enter additional comments. Dialog button 208 enables the visual representation data 70 to be stored along with the user identification 204.

In **FIG. 8C****,** a plurality of historical quality control tests and historical patient tests are provided in a "history" section of the graphical user interface available to a user of the computing device 20.

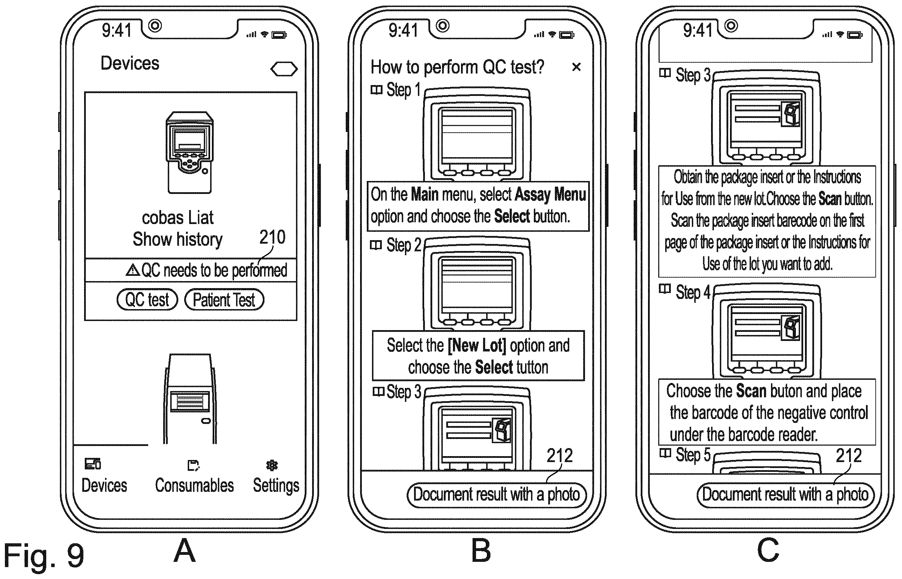

**FIGs.9A-C** schematically illustrate three exemplary graphical user interface screens for guided remote device registration and quality control testing.

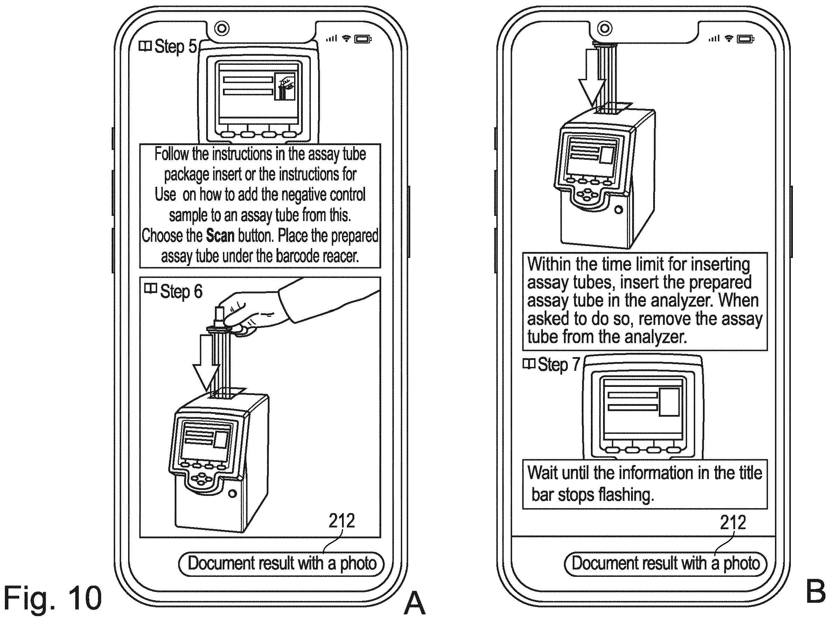

**FIGs.10A** and **10B** schematically illustrate further graphical user interface screens for guided remote quality control testing.

According to an embodiment, the predefined operation is comprised within a quality control process performed on the automated analyzer P1, and the stored evaluation data comprises a quality control result associated with the automated analyzer P1.

In this embodiment, a POC-DMS 50 and/or an automated analyzer P1 may detect that a quality control operation should be performed. For example, a time period measured by the automated analyzer P1 after a previous quality control operation may have elapsed. Accordingly, the graphical user interface of the computing device 20 notifies the user via an alert 210 that a quality control test needs to be performed, as illustrated in **FIG. 9A****.**

**FIGs.9B** and **9C** illustrate a guided quality control test procedure provided to a user of the computing device 20. A number of steps displayed on the graphical user interface guide a user of the computing device through the process of performing quality control test. The graphical user interface button 212, actuatable via a touchscreen interface, for example, automatically enables the user of the computing device 20 to document the result of the quality control test using a photograph or in other words, visual representation data 70. For example, at the end of the workflow, when the quality control test result is ready, the application hosted by the computing device 20 asks the user to take a photo of the instrument screen to document the result of the quality control or patient test.

According to an embodiment, additional attributes of the quality control or patient test can be input using the graphical user interface of the computing device 20, for example. In an embodiment, further additional attributes of the quality control or patient test can be automatically populated using method data available in the visual representation data 70, for example location, date, and time. The visual representation data 70 of the screen of the automated analyzer P1 may be analyzed by a data processing agent 32 and/or the computing device 20 to recognize a result of the test, the date or time (which should be matched with the visual representation data 70) or a unit of measurement.

According to an embodiment, the computer implemented method 60 further comprises.
prior to insertion, during, or after removal, of a test insert P1-T into the automated analyzer p1 as part of a test process:
- obtaining, via the computing device 20 comprising the camera 21, visual representation data of at least a portion P 1-TBC of a test insert P1-T for the test process;
- processing the visual representation data of the portion of the test insert P1-T to extract an identifier associated with the test process; and
- generating a logical association between the identifier associated with the test process and the visual representation data of the display interface P1-D of the automated analyzer P1, and/or the stored evaluation data associated with the predefined operation.

Accordingly, in a manner analogous to the generation of visual representation data 70 in respect of a graphical user interface of an automated analyzer P1, visual representation data of at least a portion of the test insert P1-T can be obtained before, or after, obtaining the visual representation data 70 in respect of the graphical user interface of the automated analyzer P1. This permits a user of the computing device 20 to provide a logical linkage between a visual record of the result of a test carried out using a test insert P1-T as displayed in the graphical user interface of an automated analyzer P1, and the carton and/or the portion P1-TBC of the test insert P1-T. An effect is that a user of the automated analyzer P1 can generate a guarantee that the visual representation data 70 generated by the automated analyzer P1 is generated using the test insert P1-T.

According to an embodiment, the predefined operation is comprised within a test process performed on the automated analyzer P1, and the stored evaluation data comprises a test result associated with the automated analyzer P1.

According to an embodiment, the predefined operation is a stage of a user certification process performed on the automated analyzer P1, and the stored evaluation data comprises a user certification result associated with the automated analyzer P1.

Accordingly, a user certification process obtained using an automated analyzer P1 is authenticated using the computing device 20, when the computing device 20 communicates the visual representation data 70 of the patient result to the data processing agent 32.

According to an embodiment, the predefined operation is a stage of a consumable management process, or a hardware self-test process performed on the automated analyzer P1, and the stored evaluation data comprises a consumable management result and/or a hardware self-test result associated with the automated analyzer P1.

Accordingly, a consumable management process or a hardware self-test process obtained using an automated analyzer P1 are authenticated using the computing device 20, when the computing device 20 communicates the visual representation data 70 of the patient result to the data processing agent 32.

According to an embodiment, the predefined operation is a stage of a patient result acquisition performed on the automated analyzer P1, and the stored evaluation data comprises a patient result associated with the automated analyzer.

Accordingly, patient results obtained using an automated analyzer P1 are authenticated using computing device 20, when the computing device 20 communicates the visual representation data 70 of the patient result to the data processing agent 32.

According to an embodiment, the computer implemented method 60 further comprises:
- creating an automated analyzer record in a laboratory management software application;
- selecting a type of automated analyzer P1-P7 to be associated with the created automated analyzer record from an analyzer type data store comprising a plurality of types of automated analyzer;
   wherein the data store comprises at least one image processing routine defining, for one or more predefined operations to be performed on the each of the plurality of types of automated analyzer P1-P7, a portion of a display interface P1-D of the corresponding automated analyzer associated with an outcome of the predefined operation, and/or a visual characteristic of the associated display interface of the corresponding automated analyzer associated with an outcome of the predefined operation; and
- populating the new automated analyzer record with the selected type of automated analyzer.

An operator of a computing device 20 can configure a laboratory management software application, for example, hosted by a POC-DMS 50, to represent a virtual version of a clinical laboratory complete with the point-of-care testing instruments. Accordingly, a laboratory software management application hosted by a data processing agent 32 and/or a POC-DMS 50 configured, according to an automated configuration tool, or software wizard.

Each type of automated analyzer present in the library of automated analyzers is, for example, associated with an image processing routine or specific set of imaging masks enabling a visual representation 70 of the display P1-D to have data extracted from it. For example, an image processing algorithm can be designed a priori, or a machine learning model can be trained, to extract data using an image processing mask from a visual representation 70 of a legacy analyzer.

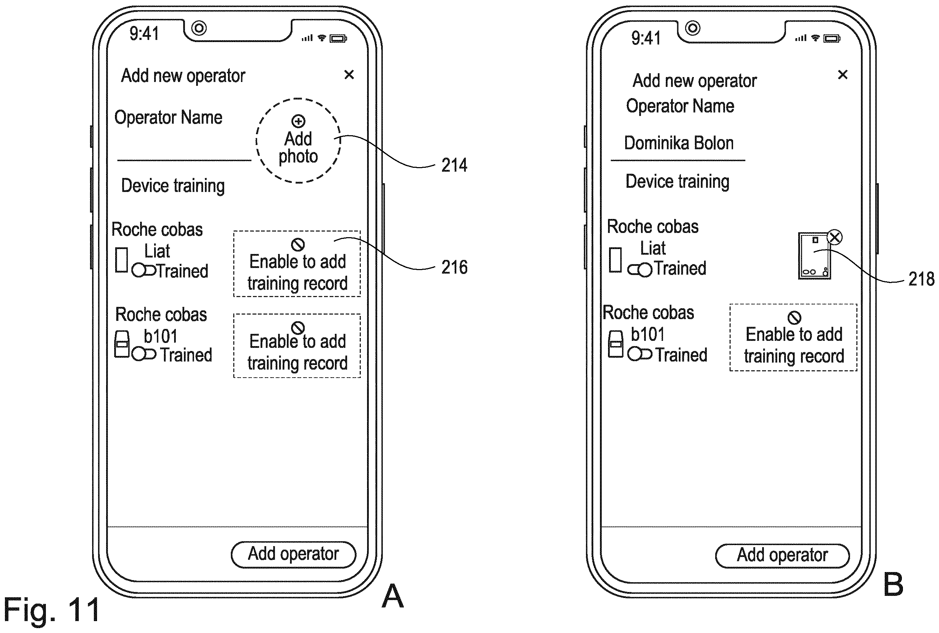

**FIGs.11A** and **11B** schematically illustrate a graphical user interface for user management.

According to an embodiment, the computer implemented method 60 further comprises:
- obtaining identification data of at least one user of the laboratory management software application;
   for each automated analyzer P1-P7 registered in the laboratory management software application, obtaining certification data of the user; and
   if a user is not certified to use an automated analyzer registered in the laboratory management software application, generating a predefined certification activity for each automated analyzer registered in the laboratory management software application; and
- monitoring the laboratory management software application for completion of each predefined certification activity for each automated analyzer registered in the laboratory management software application.

Computing device 20 can facilitate the addition of a user, or the update of an existing user, in the POC system. For example, a new record can be provided comprising the identity of an operator 214, and at least the certification status or training status of the new operator on the automated analyzers of the POC system. In the case of a training or certification non-compliance, a training course or certification step can be completed in respect of a specific automated analyzer 218.

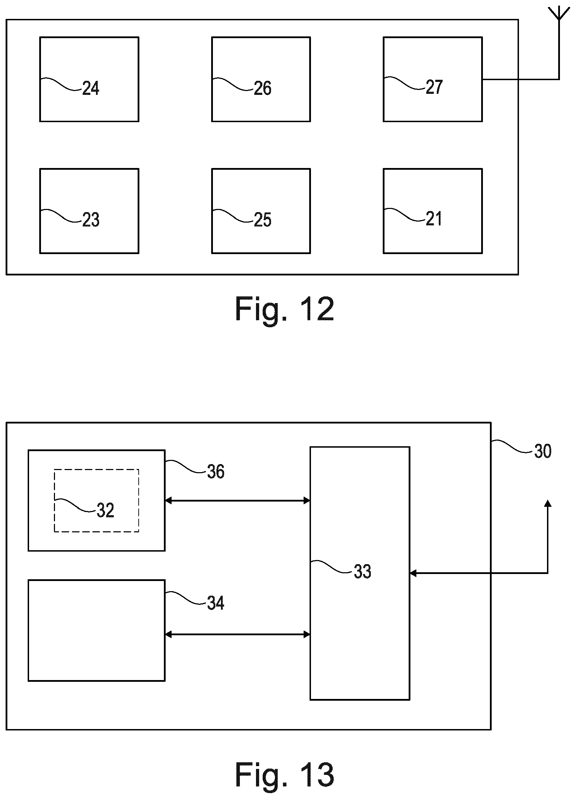

**FIG.12** schematically illustrates a computer comprising a camera.

In an embodiment, the computer 20 comprising a camera 21 is a smart phone or smart tablet comprising an integrated camera 21. According to an embodiment, the computer 20 is an Apple iPhone (TM) or a Google Android (TM)-based device.

According to an embodiment, the computer 20 comprising camera 21 may also be a personal computer comprising a web camera connected to the personal computer via USB, Bluetooth (TM), or WiFi (TM).

For example, the computer 20 comprises a camera 21. In some embodiments, more than one camera is provided. The camera is an optical sensor, such as a CCD (charge coupled device) or CMOS (complementary metal oxide semiconductor). The camera 21 may be integrated with interface electronics and an I/O (input/output) subsystem of the computer so that images and/or videos obtained of the automated analyzer are made available to a software operating environment (operating system) instantiated by the processor 24.

For example, the computer 20 comprises a user interface 23. In some embodiments, the user interface 23 is a touchscreen I/O device. The touchscreen I/O device may receive graphical display instructions from a software operating environment (operating system) instantiated by processor 24. The software operating environment may host an application configured to obtain images and/or videos of an automated analyzer. In some embodiments, the application is configured to perform image analysis and data extraction/evaluation on the images and/or videos, to obtain data contained on the display **P1-D** of the automated analyzer exemplified in **FIG. 3****.**

For example, the computer 20 comprises a processor 24. The processor is configured to obtain computer readable instructions from the data storage 26, and to instantiate a software environment on the computing device 20. In some examples, the software environment may host a specific application for obtaining and processing images of the display screen of an automated analyzer.

For example, the computer 20 comprises a location service 25. The location service may comprise a combination of hardware elements, such as an inertial measurement sensor, a magnetic compass or magnetometer, and a GPS (global positioning system) receiver. These hardware modules are communicably coupled to the other elements of the computer via an I/O subsystem. The location service may comprise software modules configured to receive location updates based on the network address of an access point being used by the computer, for example. In general, the location service can provide, to a software environment of the computer 20, an estimate of the location of the computer to within 100, 50, 20, 10, 5, or 1 metre.

For example, the computer 20 comprises data storage 26. For example, the data storage may comprise RAM, ROM, SSD, or any other suitable combination of data storage 26.

For example, the computer 20 comprises a communications interface 27. For example, the communications interface 27 is one, or more, of a WiFi (TM), Bluetooth (TM), or a 3GPP (TM) modem, or a modem for any other suitable data transmission modality.

According to an embodiment, the computer 20 is configured to capture an image and/or video comprising a field of view of the camera 21, when the camera 21 observes a display P1-D of an automated analyzer P1 after the automated analyzer P1 has completed a predetermined operation.

According to an embodiment, the computer 20 is not specially configured with application software. Native image and/or video capture software pre-installed on the computer 20 is used to capture the image and/or video from the camera 21. A standard means of image transfer installed on the operating system of the computer 20 such as email or FTP is used to communicate the image and/or video from the camera 21 to the apparatus 30. The data processing agent 32 instances on the apparatus 30 may host a specialized email server with a specific email address for incoming analyzer images and videos for analysis, for example.

The data processing agent 32 instantiated on apparatus 30 is configured to receive the email or FTP connection and must receive the image and/or video from the camera 21. Image processing according to aspects of this specification is initiated after this time. Therefore, according to this embodiment, the computer 20 is a standard device not requiring a special configuration or a customized application.

According to another embodiment, the computer 20 is configured with a software application configured to perform the method of the first aspect, or its embodiments.

FIG.13 schematically illustrates an apparatus according to the second aspect.

According to a second aspect, there is provided an apparatus 30 configured to host a data processing agent 32 for processing data from one or more automated analyzers. The apparatus comprises a communications interface 33, a data memory 34, and a processor 36 coupled to the communications interface and the data memory.

The communications interface 33 is configured to receive visual representation data 70 of at least a display interface P1-D of an automated analyzer P1 after the automated analyzer P1 has performed a predefined operation. The visual representation data 70 of the display interface comprises data associated with an outcome of the predefined operation performed by the analyzer.

The processor 36 is configured to process the visual representation data 70 to extract data relating to an outcome of the predefined operation computed by the automated analyzer P1 and comprised in the visual representation data 70 associated with the predefined operation.

The processor 36 is configured to evaluate the data associated with the predefined operation according to at least one evaluation criterion to thus generate evaluation data.

The processor 36 is configured to store the evaluation data associated with the predefined operation.

In an embodiment, the apparatus 30 stores evaluation data in the data memory 34. In another embodiment, the apparatus 30 stores the evaluation data in a datastore, or cloud service, which is external to the apparatus 30.

According to an embodiment, the data processing agent 32 is instantiated on the apparatus 30 from machine-readable instructions obtained, for example, from the datastore 34, and/or another non-transitory data storage medium or non-volatile memory.

For example, the apparatus 30 according to the second aspect is a personal computer (PC), a bare metal server, a server, or an enterprise computer providing access to a cloud instance.

The communications interface 33 may comprise one or more of a WAN or LAN adaptor. Furthermore, the communications interface 33 may comprise a unit configured to communicate with at least POC-DMS 50.

The apparatus 30 may further comprise a user interface, such as a monitor, computer mouse, and computer keyboard, to facilitate user interaction. In examples, the processor 36 may instantiate an instance of a remote desktop or Telnet application to enable remote access to the apparatus from another computer.

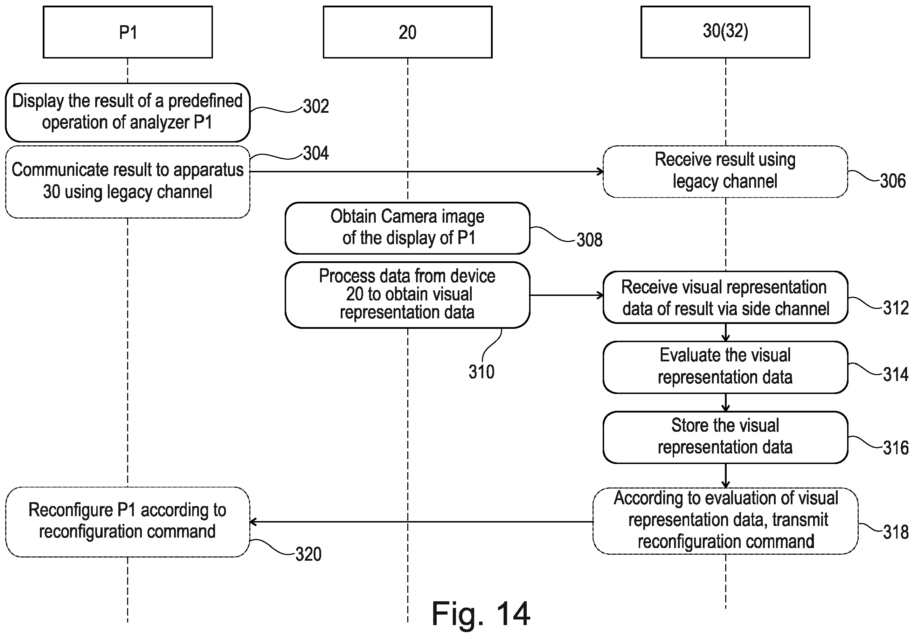

**FIG.14** schematically illustrates communication in a system according to the third aspect.

According to a third aspect, there is provided a system 10 for remote analyzer monitoring comprising:
- a computing device 20 comprising a camera 21;
- at least one automated analyzer P1-P7 of biological samples;
- an apparatus 30 configured to host a data processing agent 32 for processing data from the at least one automated analyzers P1-P7; and
- a communications network 40 configured to communicably couple the computing device 20 and the apparatus 30 via a first communication channel, and the communications network 40 is configured to communicably couple at least one automated analyzer P1-P7 and the apparatus 30 via a second communication channel distinct from the first communication channel;

wherein the computing device 20 is configured to obtain, via the computing device 20, visual representation data 70 of at least a display interface P1-D of an automated analyzer P1 after the automated analyzer has performed a predefined operation, wherein the visual representation data 70 of the display interface comprises data associated with an outcome of the predefined operation performed by the analyzer;
wherein the computing device 20 and/or the apparatus 30 are configured to process the visual representation data 70 to extract data relating to an outcome of the predefined operation computed by the automated analyzer P1 and comprised in the visual representation data 70 associated with the predefined operation;
wherein the computing device 20 and/or the apparatus 30 are configured to evaluate the data associated with the predefined operation according to at least one evaluation criterion to thus generate evaluation data; and
wherein the computing device 20 and/or the apparatus 30 are configured to store the evaluation data associated with the predefined operation.

According to an embodiment of the system 10, the computing device 20 is configured to communicate the visual representation data 70 to the apparatus via a side channel 22A, 22B of the system 10 different to the communications network 17, that communicably couples the computing device 20, the automated analyzer P1, and the apparatus 30.

At stage 302, the automated analyzer P1 displays an outcome of a predetermined operation P1-D on its display interface.

At stage 308, the user of computer 20 obtains a camera image of the display P1-D using the camera 21 of the computing device.

At stage 310, the computer 20 processes the camera image to form visual representation data 70 of the display P1-D. Processing the camera image may comprise performing image processing operations such as colour correction, automated cropping of the image, and the like. Processing the camera image may also comprise appending to the camera image meta data such as user identification data of the user who obtained the camera image, user certification data of the user who obtained the image, information concerning the type of predefined operation depicted in the display P1-D, the time and/or date at which the camera image was obtained, location data indicating where the camera image was obtained, and the like. Computer 20 communicates the visual representation data 70 via a side-channel 20B, 20C which bypasses the POC-DMS 50 of the system 10.

At stage 312, an apparatus 30 instantiating a data processing agent 32 is configured to receive the visual representation data 70 via a side-channel 20B, 20C.

At stage 314, the data processing agent 32 is configured to evaluate the visual representation data according to an evaluation criterion, to thus generate evaluation data.

At stage 316, the data processing agent 32 is configured to store the visual representation data in a local datastore 34, or in a remote data store.

According to a first option, the automated analyzer P1 can communicate 304 the result of a predefined operation performed on the analyzer P1 to the data processing agent 32 instantiated in apparatus 30 via legacy channel. For example, the automated analyzer P1 communicates the result of the predefined operation to a POC-DMS 50 using legacy signaling, and the POC-DMS 50 forwards the result of the predefined operation to the data processing agent 32 instantiated in the apparatus 30.

According to this option, the data processing agent 32 receives the result of the predefined operation 302 from the POC-DMS 50, although without contextual information such as location data, or data about the user of the automated analyzer P1. At a similar time point, the data processing agent 32 receives visual representation data 70 of the display P1-D of the automated analyzer P1 comprising the same result as forwarded from the POC-DMS 50 via the legacy channel. The visual representation data 70 comprises the same outcome of the predefined operation as contained in the result of the predefined operation sent via the legacy channel. In an embodiment, the visual representation data 70 comprises meta data enabling, for example, the identity of the user performing the predefined operation to be authenticated. In an embodiment, the visual representation data 70 comprises meta data defining a quality control status of the automated analyzer to be verified. In an embodiment, the visual representation data 70 comprises a certification status of the user performing the predefined operation to be identified.

According to another option, the data processing agent 32 may transmit 318 a reconfiguration command to the automated analyzer P1 via, for example, a legacy communication channel managed by the POC-DMS 50. Some types of automated analyzer may enable limited reconfiguration based on a received input. For example, the data processing agent 32 may, on the basis of an evaluation of the visual representation data 70, transmit a reconfiguration command that locks the automated analyzer P1. In an embodiment, the reconfiguration command is to display a specific message on the screen of the automated analyzer P1.

According to a fourth aspect, there is provided a computer program element comprising machine readable instructions which, when executed by a processor, cause the processor to perform the computer implemented method as defined in the first aspect, or an embodiment thereof.

## Claims

1. A computer implemented method (60) for remote analyzer monitoring comprising:
- obtaining (62), via a computing device (20) comprising a camera (21), visual representation data (70) of at least a display interface (P1-D) of an automated analyzer (P1) after the automated analyzer (P1) has performed a predefined operation, wherein the visual representation data (70) of the display interface (P1-D) comprises data associated with an outcome of the predefined operation performed by the automated analyzer (P1);
- processing (64) the visual representation data to extract data (74) relating to an outcome of the predefined operation computed by the automated analyzer (P1) and comprised in the visual representation data (70) associated with the predefined operation;
- evaluating (66) the data associated with the predefined operation according to at least one evaluation criterion to thus generate evaluation data (76); and
- storing (68) the evaluation data associated with the predefined operation.

2. The computer implemented method (60) according to claim 1, further comprising:
obtaining, via a user interface of the computing device, an identifier (72) of a user performing the predefined operation using the automated analyzer (P1); and
generating at least one logical association between the identifier (72) of the user and the visual representation data (70) and/or evaluation data (74);
wherein the at least one evaluation criterion is a comparison (71) of the identifier (72) of the user and a user record (81) of the data (74) relating to an outcome of the predefined operation.

3. The computer implemented method (60) according to claims 1 or 2, further comprising:
obtaining location data (79) of the automated analyzer (P1) using a location service (25) of the computing device (20); and
generating a logical association between the location data (79) of the automated analyzer (P1) and the location data comprised in the evaluation data (80);
wherein the at least one evaluation criterion is a comparison of location data (80) of the user and a user record (81) of the data (74) relating to an outcome of the predefined operation.

4. The computer implemented method (60) according to one of the preceding claims, further comprising:
displaying, on a user interface (23) of the computing device (20), at least one graphical instruction associated with the predefined operation to be performed on the automated analyzer (P1); and
receiving, via the user interface (21) of the computing device (20), a confirmation that the user intends to obtain the visual representation data (70), wherein the visual representation data (70) comprises a representation of a display interface of the automated analyzer when the automated analyzer has previously performed a predefined operation according to the at least one graphical instruction associated with the predefined operation.

5. The computer implemented method (60) according to claim 4, further comprising:
generating an identifier of the predefined operation to be performed on the automated analyzer (P1) as defined in the at least one graphical instruction; and
after obtaining the visual representation data (70) comprising the representation of the display interface (P1-D) of the automated analyzer (P1):
generating a logical association between the identifier of the predefined operation and the visual representation data (70), and/or the stored evaluation data associated with the predefined operation; and
storing the identifier of the predefined operation.

6. The computer implemented method (60) according to one of the preceding claims, further comprising:
detecting a predefined change of the display interface (P1-D) of the automated analyzer indicating that the automated analyzer (P1) has completed the predefined operation; and
upon detecting the predefined change of the display interface (P1-D), automatically obtaining the visual representation data (70) using the camera (21) of the computing device (20).

7. The computer implemented method (60) according to one of the preceding claims, further comprising:
prior to insertion, during, or after removal, of a test insert (P1-T) into the automated analyzer (p1) as part of a test process:
obtaining, via the computing device (20) comprising the camera (21), visual representation data of at least a portion (P1-TBC) of a test insert (P1-T) for the test process;
processing the visual representation data of the portion of the test insert (P1-T) to extract an identifier of the test process; and
generating a logical association between the identifier of the test process and the visual representation data of the display interface (P1-D) of the automated analyzer (P1), and/or the stored evaluation data associated with the predefined operation.

8. The computer implemented method (60) according to one of the preceding claims,
wherein the predefined operation is comprised within a quality control process performed on the automated analyzer (P1), and the stored evaluation data comprises a quality control result associated with the automated analyzer (P1); and/or
wherein the predefined operation is comprised within a test process performed on the automated analyzer (P1), and the stored evaluation data comprises a test result associated with the automated analyzer (P1); and/or
wherein the predefined operation is a stage of a user certification process performed on the automated analyzer (P1), and the stored evaluation data comprises a user certification result associated with the automated analyzer (P1); and/or
wherein the predefined operation is a stage of a consumable management process, or a hardware self-test process performed on the automated analyzer (P1), and the stored evaluation data comprises a consumable management result and/or a hardware self-test result associated with the automated analyzer (P1); and/or
wherein the predefined operation is a stage of a patient result acquisition performed on the automated analyzer (P1), and the stored evaluation data comprises a patient result associated with the automated analyzer.

9. The computer implemented method (60) according to one of the preceding claims, further comprising:
creating an automated analyzer record in a laboratory management software application;
selecting a type of automated analyzer (P1-P7) to be associated with the created automated analyzer record from an analyzer type data store comprising a plurality of types of automated analyzer;
wherein data store comprises at least one image processing routine defining, for one or more predefined operations to be performed on the each of the plurality of types of automated analyzer (P1-P7), a portion of a display interface (P1-D) of the corresponding automated analyzer associated with an outcome of the predefined operation, and/or a visual characteristic of the associated display interface of the corresponding automated analyzer associated with an outcome of the predefined operation; and
populating the new automated analyzer record with the selected type of automated analyzer.

10. The computer implemented method (60) according to one of the preceding claims, further comprising:
transmitting a reconfiguration command to the automated analyzer (P1) according to the evaluation data associated with the predefined operation; and
reconfiguring the automated analyzer (P1) based on the reconfiguration command, wherein the reconfiguration command is optionally a software lock of the automated analyzer (P1), or a requirement for a user for perform a quality control or certification workflow.

11. The computer implemented method (60) according to one of claims 9 or 10, further comprising:
obtaining identification data of at least one user of the laboratory management software application;
for each automated analyzer (P1-P7) registered in the laboratory management software application, obtaining certification data of the user; and
if a user is not certified to use an automated analyzer registered in the laboratory management software application, generating a predefined certification activity for each automated analyzer registered in the laboratory management software application; and
monitoring the laboratory management software application for completion of each predefined certification activity for each automated analyzer registered in the laboratory management software application.

12. An apparatus (30) configured to host a data processing agent (32) for processing data from one or more automated analyzers; comprising:
- a communications interface (33);
- a data memory (34); and
- a processor (36) coupled to the communications interface and the data memory;
wherein the communications interface (33) is configured to receive visual representation data (70) of at least a display interface (P1-D) of an automated analyzer (P1) after the automated analyzer (P1) has performed a predefined operation, wherein the visual representation data (70) of the display interface comprises data associated with an outcome of the predefined operation performed by the analyzer;
wherein the processor (36) is configured to process the visual representation data (70) to extract data relating to an outcome of the predefined operation computed by the automated analyzer (P1) and comprised in the visual representation data (70) associated with the predefined operation;
wherein the processor (36) is configured to evaluate the data associated with the predefined operation according to at least one evaluation criterion to thus generate evaluation data; and
wherein the processor (36) is configured to store the evaluation data associated with the predefined operation.

13. A system (10) for remote analyzer monitoring comprising:
- a computing device (20) comprising a camera (21);
- at least one automated analyzer (P1-P7) of biological samples;
- an apparatus (30) configured to host a data processing agent (32) for processing data from the at least one automated analyzer (P1-P7); and
- a communications network (40) configured to communicably couple the computing device (20) and the apparatus (30) via a first communication channel, and the communications network (40) is configured to communicably couple at least one automated analyzer (P1-P7) and the apparatus (30) via a second communication channel distinct from the first communication channel;
wherein the computing device (20) is configured to obtain, via the computing device (20), visual representation data (70) of at least a display interface (P1-D) of an automated analyzer (P1) after the automated analyzer has performed a predefined operation, wherein the visual representation data (70) of the display interface comprises data associated with an outcome of the predefined operation performed by the analyzer;
wherein the computing device (20) and/or the apparatus (30) are configured to process the visual representation data (70) to extract data relating to an outcome of the predefined operation computed by the automated analyzer (P1) and comprised in the visual representation data (70) associated with the predefined operation;
wherein the computing device (20) and/or the apparatus (30) are configured to evaluate the data associated with the predefined operation according to at least one evaluation criterion to thus generate evaluation data; and
wherein the computing device (20) and/or the apparatus (30) are configured to store the evaluation data associated with the predefined operation.

14. The system (10) for remote analyzer management according to claim 13,
wherein the computing device (20) is configured to communicate the visual representation data (70) to the apparatus via a side channel (22A, 22B) of the system (10) different to the communications network (17) that communicably couples the computing device (20), the automated analyzer (P1), and the apparatus (30).

15. A computer program element comprising machine readable instructions which, when executed by a processor, cause the processor to perform the computer implemented method as defined in one of claims 1 to 11.
